# EUROPEAN PATENT APPLICATION

(11) **EP 4 763 058 A1**
(43) Date of publication of application: **24.06.2026**
(21) Application number: 25225961.9
(22) Date of filing: 19.12.2025
(51) Int. Cl.: A61B 5/00, A61B 5/022, G16H 10/20, G16H 20/10, G16H 40/63, G16H 40/67, G16H 50/30, G16H 80/00

(54) **CONTEXTUAL REMOTE PATIENT MONITORING SYSTEM**

(30) Priority: 20.12.2024 FR 2414846
(71) Applicant: Withings, 92130 Issy-Les-Moulineaux (FR)
(72) Inventor: Brini, Younes, 92130 Issy-les-Moulineaux (FR); Attia, Samuel, 92130 Issy-les-Moulineaux (FR)
(74) Representative: Withings IP

(57) **Abstract**

The invention relates to a remote patient monitoring - RPM - system capable of collecting enriched physiological data comprising blood pressure data enriched with contextual data comprising a question displayed to the patient and a predetermined response to the question received from the patient, and adapting the notification level at a remote monitoring interface based on the enriched physiological data.

## Description

### Technical field

The invention relates to remote patient monitoring system, known as RPM system. Those systems usually involve a measuring device, such as a BP (Blood Pressure) monitor, embedding a sensor capable of measuring physiological data of a user (also called a patient), and wirelessly connected to a network, and a monitoring device, embedding a user interface and/or a data management flow, so that a care team may monitor the patient remotely.

The goal of RPM system is to continuously monitor a patient's vitals, such as his or her weight, blood pressure, blood oxygen saturation, etc.

### Background of the invention

In RPM system, healthcare professionals remotely monitor patients using measuring devices. In the field of blood pressure, high BP data usually triggers a routine which requires the healthcare professionals (doctor or nurse) to reach out to the patient. However, some BP data are imperfectly taken and do not need immediate attention as they are external factors diminishing the criticality of the BP data, and some BP data are correctly high but not critical, as the criticality may depend on other factors.

Documents EP1726257, WO2017/089171, US20240172990A1, US2013267795, WO2019/200158, US 8438038 disclose various solutions, some of them for RPM systems, some of them embedding user surveys or questions to enrich the measurement.

Withings^{™} provides body scales, blood pressure monitors (notably the BPM Pro 2^{™}) and other devices suitable for RPM systems.

There is a need to provide improved RPM systems that will streamline the RPM process for professionals by decreasing the number of false positives, that are cases for which an intervention of the health professional is not required, and/or by better understand what's behind a BP data.

### Summary of the disclosure

The present disclosure is about a remote patient monitoring system (called "RPM system"), involving a user (or patient) and a professional (e.g., a physician, a nurse and any other professional) remotely taking care of the user thanks to connected physiological devices.

An object of the disclosure is a remote patient monitoring, RPM, system including a physiological measurement device comprising a physiological sensor, a user interface comprising a display and input hardware, comprising a wireless communication module, the RPM system further including a remote monitoring interface, configured to run on a remote monitoring device, wherein the remote monitoring device and the physiological measurement device are two distinct devices. The physiological measurement device is configured to send data to the remote monitoring interface using the wireless communication module. The physiological measurement device is configured to
- obtain physiological data using the physiological sensor,
- display on the display of the physiological measurement device a question with a plurality of

predetermined responses, wherein the question and the plurality of predetermined responses form a plurality of contextual data
- receive a predetermined response selected by the user using the input hardware of the BP monitor, wherein the question and the selected predetermined response form selected contextual data,
- attribute a notification level to the physiological data using the physiological data and the selected contextual data, wherein the notification level is chosen amongst a plurality of available notification levels
- display at the remote monitoring interface a display mode based on the attributed notification level.

Another aspect of the disclosure is a remote patient monitoring, RPM, system including:
- a blood pressure, BP, monitor, comprising: a case, a user interface mounted on the case, with a display and input hardware, a blood pressure, BP, sensor configured to generate blood pressure data about the use, a wireless communication module,
- a remote monitoring interface, configured to run on a remote monitoring device, wherein the remote monitoring device and the BP monitor are two distinct devices.

The BP monitor is configured to send data to the remote monitoring interface using the wireless communication module.

The RPM system is configured to:
- obtain BP data using the BP sensor of the BP monitor,
- display on the display of the BP monitor a question with a plurality of predetermined responses, wherein the question and the plurality of predetermined responses form a plurality of contextual data,
- receive a predetermined response selected by the user using the input hardware of the BP monitor, wherein the question and the selected predetermined response form selected contextual data,
- attribute a notification level to the BP data using the BP data and the selected contextual data, wherein the notification level is chosen amongst a plurality of available notification levels,
- display at the remote monitoring interface a display mode based on the attributed notification level.

Thanks to these RPM systems, contextual data are used to change the notification level of the physiological measurement thereby helping the professional taking care of the patient to better address the actual critical physiological data. In the BP monitor case, having a fully-integrated device which takes the measurement, displays questions and receives the user input for the question, during a same measurement session, enables to actually receive user input regarding the BP measurement. The BP monitor is easy to use, even for people non familiar with it or elderly people.

The following paragraphs will present additional features of the RPM systems disclosed above.

In an embodiment, the plurality of available notification levels includes between 3 and 5 notification levels.

In an embodiment, the remote monitoring interface includes a plurality of display modes, wherein each display mode includes a dedicated layout corresponding to a notification level, such that the display modes display physiological data (e.g., BP data), for which contextual data are available, differently in function of the notification level. In particular, identical physiological data (e.g., BP data) with different selected contextual data which have led to the physiological data (e.g., BP data) having different notification levels have different display modes, and different display modes display the physiological data differently.

In an embodiment, the display mode includes a notification inbox, updated based on the attributed notification level.

In an embodiment, physiological data (e.g., BP data) is an average of several physiological data values (e.g., BP data value).

In an embodiment, displaying the question is performed in response to determining that the physiological data (e.g., BP data) verify a health condition. For example, the physiological data are under or above a threshold. The health condition may be editable (e.g., added, changed, updated, etc.) via the remote monitoring interface, such that the professional can customize the health condition to better suit his or her practice (specialty, disease, patients, etc.), in particular user by user.

In an embodiment, the display mode includes displaying the physiological data (e.g. BP data) along with the selected contextual data.

In an embodiment, the RPM further includes a server and the wireless communication module of the physiological device (e.g., the BP monitor) sends to the server the physiological data (BP data) and the selected contextual data and the remote monitoring interface receives from the server the physiological data (e.g., BP data), the selected contextual data and the attributed notification level.

In an embodiment, attributing the notification level includes querying a correspondence database using the physiological data (e.g., BP data) and the selected contextual data, wherein the correspondence database links a plurality of notification levels or of notification level variations to ranges values for the BP data and the contextual data. Notification level variations are to be applied to a preexisting notification level depending only on the physiological data (e.g., BP data)

In an embodiment, the correspondence database is stored on the server and the attribution is performed by the server.

In an embodiment, the correspondence database is stored on the BP monitor and the attribution is performed by the BP monitor.

In an embodiment, the RPM system further includes a remote monitoring device configured to run the remote monitoring interface, wherein the correspondence database is stored in the remote monitoring device.

In an embodiment, the correspondence database is editable via the remote monitoring interface, such that the professional can customize the correspondence database to better suit his or her practice (specialty, disease, patients, etc.), in particular user by user.

In an embodiment, the wireless communication module is a cellular module.

Another object of the disclosure is a remote patient monitoring method, using any of the RPM systems as presented above, the method comprising:
- obtaining physiological data (e.g., BP data) using the physiological sensor (BP sensor of the physiological measurement device (e.g., BP monitor),
- display on the display a question with a plurality of predetermined responses, wherein the question and the predetermined response form predetermined contextual data,
- receive a predetermined response selected by the user using the input hardware, wherein the question and the selected predetermined response form selected contextual data,
- attribute a notification level to the physiological data (e.g., BP data) using the physiological data (e.g., BP data) and the selected contextual data, wherein the notification level is chosen amongst a plurality of available notification levels,
- display at the remote monitoring interface a display mode based on the attributed notification level.

Another object of the disclosure is a server or a method from the server point of view, for any of the RPM system as presented above:
- the server sends to the physiological measurement device (e.g., BP monitor) a question database, the question database including at least one question and a plurality of predetermined responses for each question, wherein the question and the predetermined response form contextual data,
- the server sends to the physiological measurement device (e.g., BP monitor) a health condition, wherein the health condition is to be checked with the physiological data (e.g., BP data),
- the server receives from the physiological measurement device (e.g., BP monitor), physiological data (e.g., BP data) with a predetermined response of a question, wherein the question and the selected predetermined response form selected contextual data.
- the server attributes a notification level to the physiological data (e.g., BP data), for example using a correspondence database as disclosed previously that is stored on the server,
- finally the server sends out to the remote monitoring interface the physiological data (e.g., BP data), the selected contextual data along with the attributed notification level.

The additional features disclosed previously also applicable to the server, when relevant.

### Description of the drawings

Other features will be visible on the following non limitative drawings:
[Fig. 1] Figure 1 shows a remote patient monitoring, RPM, system, according to an embodiment of the disclosure,
[Fig. 2] Figure 2 shows a BP monitor according to an embodiment of the disclosure,
[Fig. 3] Figure 2 shows a remote monitoring interface run on a monitoring device according to an embodiment of the disclosure,
[Fig. 4] Figure 4 shows a server according to an embodiment of the disclosure,
[Fig. 5] Figure 5 shows BP data obtained and displayed by the BP monitor according to an embodiment of the disclosure,
[Fig. 6] Figure 6 shows examples of questions displayed by the BP monitor according to an embodiment of the disclosure,
[Fig. 7] Figure 7 shows an example of a display mode with notification levels on the remote monitoring interface according to an embodiment of the disclosure,
[Fig. 8] Figure 8 shows an example of a display mode with enriched BP data on the remote monitoring interface according to an embodiment of the disclosure,
[Fig. 9] Figure 9 shows an correspondence database according to an embodiment of the disclosure,
[Fig. 10] Figure 10 shows a RPM method performed by a RPM system, according to an embodiment of the disclosure,
[Fig. 11] Figure 11 shows a RPM method performed by the server, according to an embodiment of the disclosure.

### Detailed description

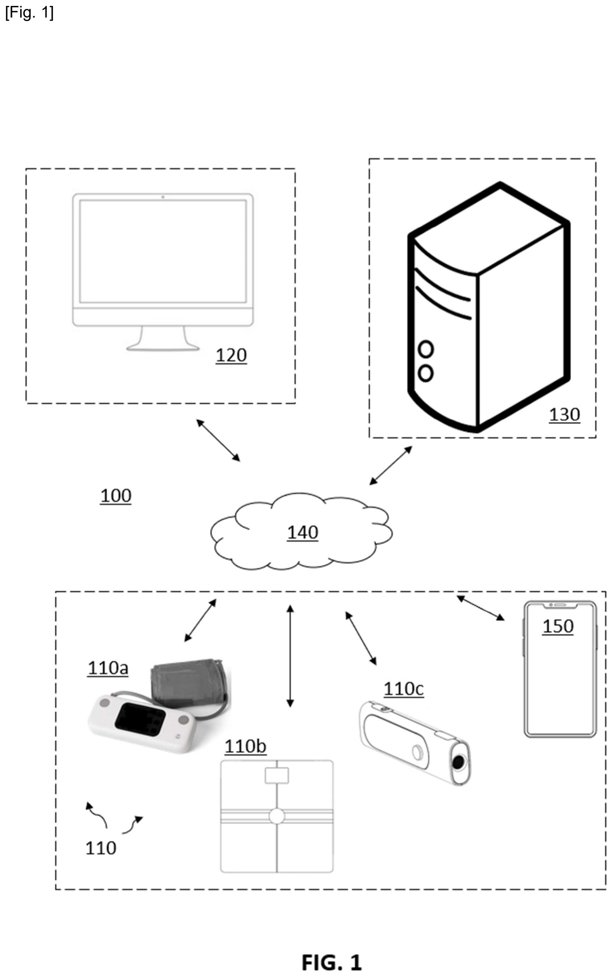

**Figure 1** illustrates a remote patient monitoring system 100, called RPM system 100. The RPM system includes at least one physiological measurement device 110 (here a blood pressure monitor 110a, called BP monitor, and/or a body scale 110b, and/or a multiscope 110c (such as disclosed in EP24217550 or devices called BeamO^{™}), but could comprise other devices, such as those disclosed in EP4386383 to Withings^{™}, or devices U-Scan^{™} developed by Withings^{™}), a remote monitoring interface 120, a server 130 and a communication network 140. The RPM system 100 may comprise also a mobile device 150, such as a smartphone or smartwatch. The remote monitoring interface 120 is configured to run on a remote monitoring device (not shown on Figure 1), which is remotely arranged from the BP monitor 110a. The mobile device 150 may be configured to run a close monitoring interface, suitable for the user.

The RPM system 100 is used by various people, who are defined as follows: the user of the BP monitor 110a is referred to as a patient and the user of the monitoring device 140 is referred to as a professional, since he or she is a healthcare professional, such as a nurse or a physician. The professional and the user in a RPM system are usually not in the same room, but usually at their respective work or living locations, except for a specific appointment, such as a follow checkup.

In a typical situation, the BP monitor 110a measures blood pressure data (BP data) on the patient and sends the BP data to the server 130 via the communication network 140, then the server 130 sends the BP data or a data obtained from the BP data to the remote monitoring device 120, via the communication network 140. As it will be disclosed in more details, the RPM system 100 of the present disclosure also allows adding contextual information pertaining to the BP data. The close monitoring interface of the mobile device 150 may allow the user to see the BP data or any related-data.

The RPM system 100 is aimed at gathering blood pressure data on the patient, via the BP monitor 110a, and at providing them to the professional, via the monitoring device 120.

The BP monitor 110a, the monitoring device 120 and the server 130 all exchange data using the communication network 140, which may be a hybrid network (ethernet, Wi-Fi, 3G, 4G, 5G, etc.).

### The BP monitor 110a

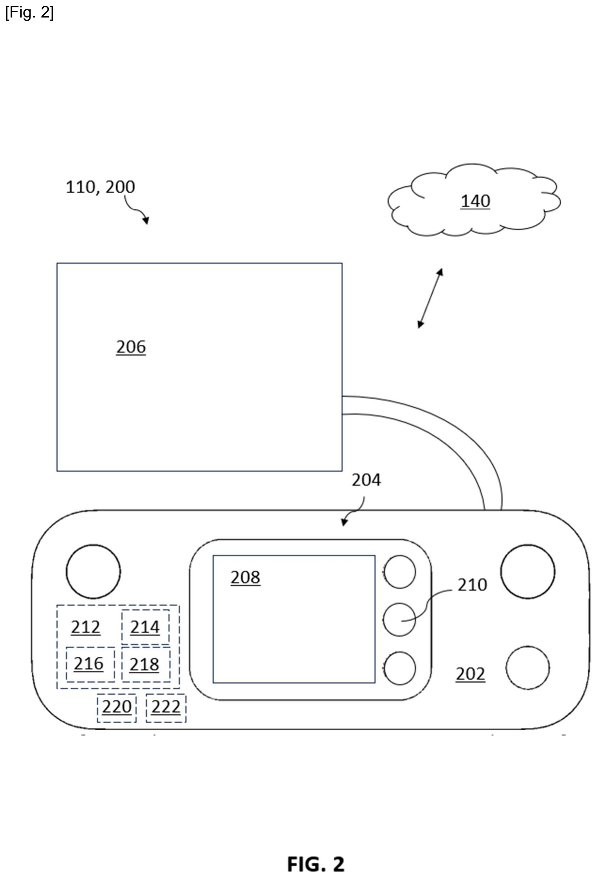

**Figure 2** illustrates a BP monitor 200 corresponding to the BP monitor 110a of Figure 1. The BP monitor 200 includes a case 202, a user interface 204 mounted on the case 202 and a blood pressure sensor 206. The user interface 204 includes a display 208 and an input hardware 210. The display 208 may be a screen, as visible on Figure 2, or a speaker. From the BP monitor 200 standpoint, the display 208 is aimed at providing information to the user while the input hardware 210 is aimed at receiving information from the user. The input hardware 210 may comprise buttons, as illustrated. As the BP monitor 200 is targeting elder people, the simpler the input hardware 210 the better. Alternatively or complementarily, the input hardware 210 may comprise a tactile screen.

The blood pressure sensor 206 is shown as a cuff on Figure 2 but the blood pressure sensor 206 typically includes the cuff, a pump (connected to the cuff via a flexible pipe) and a pressure sensor configured to generate pressure data in the cuff.

As schematically shown on Figure 2, the BP monitor 200 includes a control unit 212, comprising a processor 214, a memory 216 and a I/O (in/out) interface 218. The memory 216, which includes storage, is configured to store instructions, which, when executed by the process, allows the BP monitor 200 to notably take BP measurement, display information on the screen, receive user input. The I/O interface 218 manages the interactions of the control unit 212 with the other components. A communication module 220 enables the BP monitor 200 to exchange data, typically in a wireless manner with the communications network 140. A battery 222 stores energy to allow the BP monitor 200 to work without being electrically plugged.

In an embodiment the communication module 220 is a cellular module capable of exchanging data on a cellular network of the communication network, so that no mobile device 150 is required to forward any data generated by the BP monitor 110a.

The BP monitor 110a may be shared by several users. To that end, each user has a user account. Data generated for a same user is associated with the same user account.

### The remote monitoring interface 120

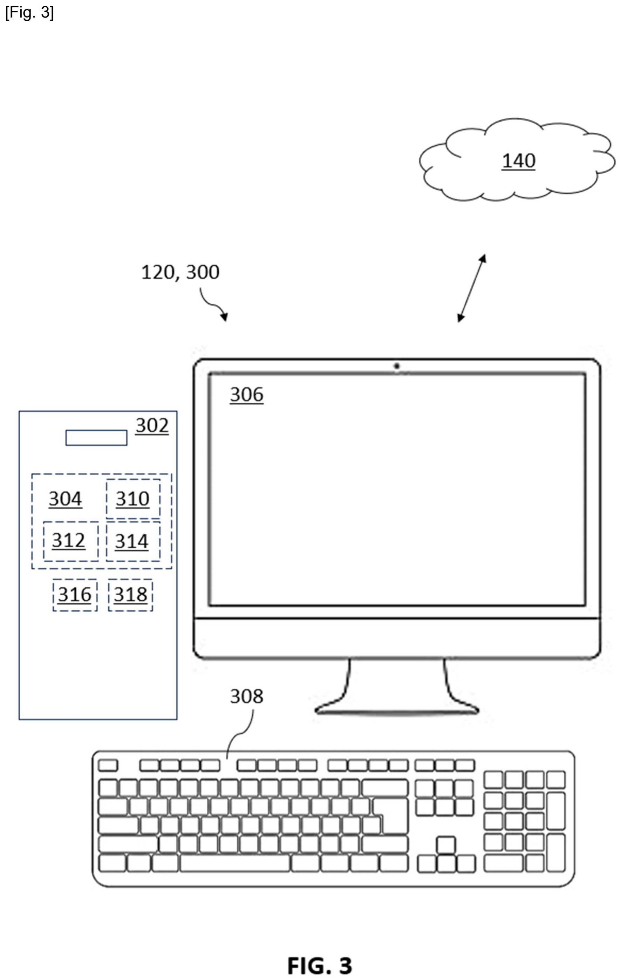

**Figure 3** illustrates a remote monitoring interface 300 corresponding to the remote monitoring interface 120 of Figure 1. The remote monitoring device interface 300 can be run by a monitoring device 302 using a control unit 304 capable of processing data, including receiving data from the server 130 and displaying data. The remote monitoring interface 300 is configured for permitting an interaction with the professional. To that end the remote monitoring interface 300 may include a display 306, such as a screen, and a hardware input 308, such as a keyboard and/or a mouse, or a trackpad. The remote monitoring device 302 is typically a personal computer (e.g., a laptop).

The remote monitoring interface 300 is typically a software-based interface, which can be deployed as an app, for example accessible on an Appstore, or a webapp or a web page accessible from a navigator. The remote monitoring interface 300 is generally provided as a SaaS (software as a service).

As schematically shown on Figure 3, the control unit 304 comprises a processor 310, a memory 312 and a I/O (in/out) interface 314. The memory 312, which includes storage, is configured to store instructions, which, when executed by the process, allows the monitoring device 302 to notably display information on the display 306 and receive user input via the hardware input 308. The I/O interface 314 manages the interactions of the control unit 302 with the other components. A communication module 316 enables the monitoring device 302 to exchange data with the communication network 140. A battery 318 may store energy to allow the monitoring device to work without being electrically plugged.

To qualify as a RPM system 100, the remote monitoring interface 120 is generally not activated on the BP monitor 110a or the mobile phone of the user, but only on the monitoring device 302 of the professional.

The remote monitoring interface 120, 300 gives access to the professional to data from various user accounts, notably (and only) the user accounts of the user which are under a RPM program by the professional.

### The server 130

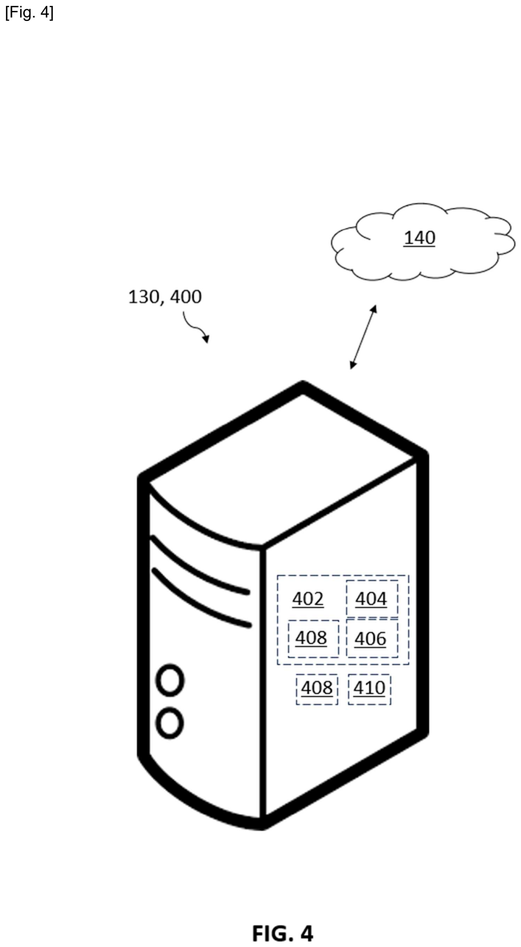

**Figure 4** illustrates a server 400 corresponding to the server 130 of Figure 1. The server 130 is a remote hardware unit configured to receive, store and process data received from the BP monitor 110a and to send the BP data or any related data to the remote monitoring interface 120. Conversely, the server 130 is also configured to receive, store and process data received from the remote monitoring interface 120 and to send the data or any related data. The server 130 is typically remotely located from the BP monitor 110a and the remote monitoring interface 120. The server 130 may be located in a datacenter, for example a datacenter provided by a datacenter provider or by the BP monitor provided or by the hospital data center of the professional.

The server 400 may store the user accounts (with all the associated data) in a centralized manner and acts as a cloud backup.

As schematically shown on Figure 4, the server 400 includes a control unit 402, comprising a processor 404, a memory 406 and a I/O (in/out) interface 408. The memory 406, which includes storage, is configured to store instructions, which, when executed by the process, allows the server 400 to query a correspondence database which will be disclosed in more detail later on. The I/O interface 408 manages the interactions of the control unit 402 with the other components. A communication module 408 enables the BP monitor 200 to exchange data, typically in a wired manner with the communications network 140.

The server 130, 400 may include a plurality of servers, for example a first server provided by the BP monitor provider and a second server provided by the professional's facilities, the first and the second server exchanging data via API (application programming interface), or a server storing a user EHR (electronic health records). From the RPM system of the present invention, any combination of those servers is considered as the server.

### The RPM system

The description will now explain in more detail how the RPM system 100 works. In particular, the RPM system 100 is aimed at providing contextual information to the BP data so that the RPM system may adapt the display of the BP data at the remote monitoring interface 120. This provides helpful assistance to the professional in sorting the users that need immediate support because of their BP data from users whose BP data have been negatively influenced by their behavior (lack of medication, physical effort before the measurement, etc.).

All data of a same user is associated with a user account.

### BP data

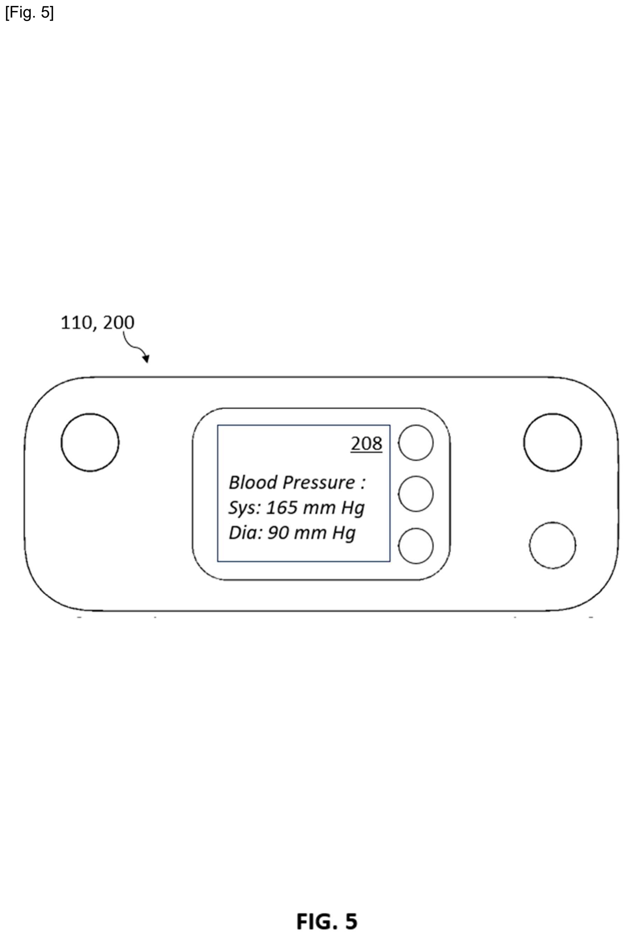

The BP monitor 110a is configured to obtain BP data from the user. This is well known and will not be disclosed in more detail. The BP data may be displayed on the display 208, as shown on Figure 5. However, as the BP data may be influenced by external factors, as disclosed previously, the RPM system requests contextual information.

In an embodiment, the BP data are an average value of several measurements, either taken successively in the same session (for example three successive BP measurements with a one-minute rest between), or taken on different days.

### Questions

To gather contextual information, the BP monitor 110a is configured to display, on the display 208, one or more questions for the user. Typically, the question is displayed after the BP data has been displayed.

In an embodiment, the question is displayed regardless of the value of the BP data; that is to say the BP data are not checked. This means that contextual information will be generated for every BP data, and thus for each BP measurement taken by the user. This allows the professional to gather much more intelligence about the user's health but this makes the BP measurement process more cumbersome for the user, who might lose patient and stop taking BP measurement.

In another embodiment, the question is displayed if the BP data verifies a health condition. This avoids generating contextual information for BP data that are considered for example as healthy. The health condition may be a BP data range value, for example a BP systolic pressure value or range. The health condition may be that under 119 mm Hg for the BP systolic pressure, no question is displayed by the BP monitor 110a.

The health condition may be tailored for each user, notably by the professional, using the remote monitoring interface 120.

The health condition enables to avoid interacting with the user when it is not necessary and to avoid generating contextual data for the professional when it is not necessary either. The goal of the RPM system of the present disclosure is to streamline the process to improve the quality of the remote monitoring by the professional. Therefore any "noise" has to be avoided.

To each question corresponds a limited number of responses that the user can select on the display using the input hardware, called predetermined responses.

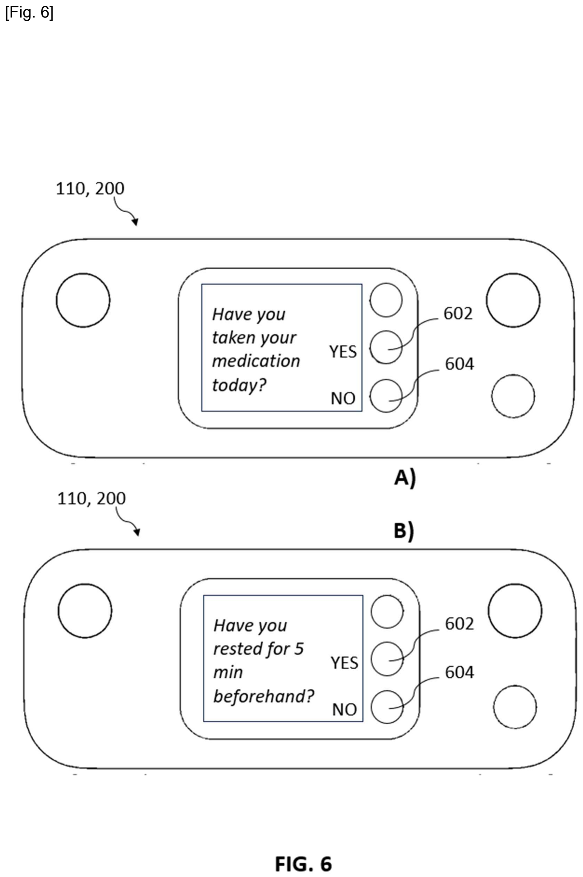

In an embodiment, the question is a yes/no question and one button is attributed to YES and another button is attributed to NO. This makes the BP monitor 110a particularly simple to use, notably for elder patients. Examples A) and B) on **Figure 6** show yes/no question on the BP monitor 110a, with buttons 602, 604 attributed to respectively YES and NO.

In an embodiment, the question is a series of successive sub-questions and the possible responses for each question is a number, so that the predetermined responses for the question (here the series of successive sub-questions) is also number, for example all the possible value for sum of all the numbers answered to the series of successive question or all the possible value of average thereof.

The questions and the predetermined responses for each question are stored in a question database. Each question with the plurality of predetermined responses form contextual data that can be selected by the user using the hardware input 210. The question and the selected predetermined question form selected contextual data.

The following list provides non-limiting examples of questions with associated responses:
have you taken your medication today? Yes/No; have you rested for 5min before taking your BP? Yes/No; did you sleep well? Yes/No; have you eaten a particularly salty meal? Yes/No. Other examples of questions are provided in the documents cited in the introduction.

The question database is stored at the BP monitor 110a, while a master question database is stored at the server 130 and a fraction of it (the question database thus) is downloaded from time to time by the BP monitor 110a. The master question database may be updated, notably through the remote monitoring interface 120, by the professional, for example to draft specific questions for certain users. The master question database and the question database may be identical. However, should there be several health issues to be addressed with different needs as for contextual data, the master question database will be populated with many more questions.

The measured BP data and the selected contextual data are associated together and are sent to the server 130.

The notification level and the display modes

One of the issues of the notification levels is to manage the professional's attention span and the professional's time to address users in need. However, some BP data may be abnormally high but may be less critical than others, notably because the user's recent behavior has tended to increase the BP data value. For example a plurality of notification levels is available for managing the BP data at the remote monitoring interface 120.

For example, there may be three levels of notifications levels: green, yellow and red. A green level means that the professional does not need to be alerted, while the yellow level means that the professional should have a look at the BP data and, finally, the red level means that the professional needs to look at the BP data as soon as possible. A fourth level of notification may be implemented, for cases that are not urgent but need to be looked at: for example, a BP data for a patient suffering from hypertension that is in the normal BP value ranges.

Based on the notification level, the remote monitoring interface 120 may display various display modes. The various display modes are to be assessed for BP data with contextual data, meaning that identical BP data with different selected contextual data which have led to the BP data having different notification levels have different display modes. Said otherwise, different display modes display the BP data differently.

In an embodiment, the layout between various display modes varies upon the notification level. There is a predetermined number of display modes, corresponding to the number of notification levels for example.

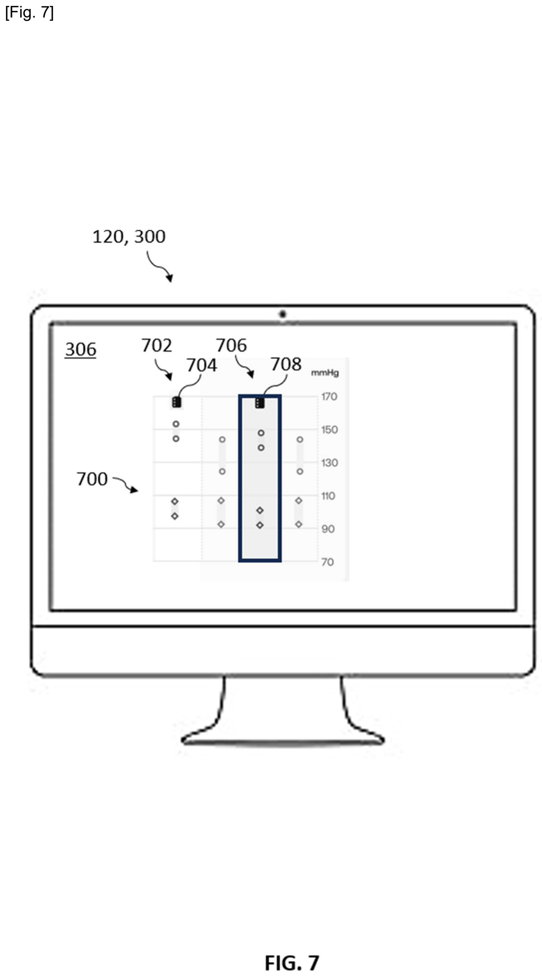

For example, BP data with a red notification level appear with a flag, while BP data with a green notification level does not stand out. Figure 7 illustrates an example of display modes 800, in which measurement 702 and measurement 706, both high BP data with contextual data, do not appear in the same way because measurement 702 includes contextual data (see icon 704) about the BP data that triggered a lower notification level than measurement 706 which includes contextual data (see icon 708) about the BP data that triggered a higher notification level. A higher notification level implies a higher emergency character than a lower notification level. Measurement 706 has for example a colored background while measurement 702 does not have one. Just by looking at the display modes, the professional is informed that measurement 706 presents a higher emergency character than measurement 702.

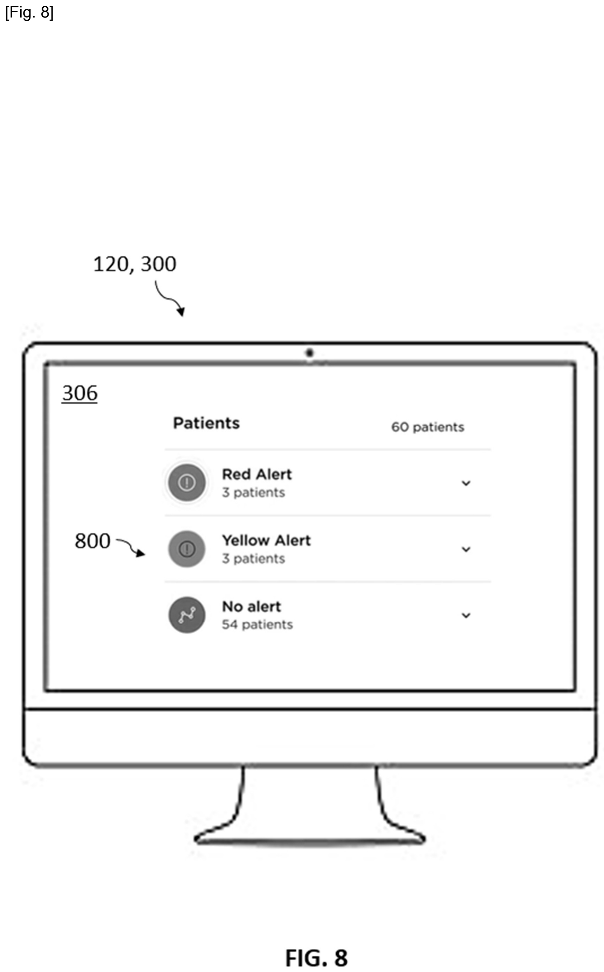

In an embodiment, the layout between various display modes is standard for all notification levels but there is a notification inbox, which is updated based on the attributed notification level. Figure 8 illustrates an example of a notification inbox 800, in which the green level is called "no alert", yellow level is called "yellow alert" and red level is called "red alert".

In an embodiment, the remote monitoring interface 120 displays the enriched BP data, that is to say that the selected contextual data are displayed along with the BP data. The RPM system 100 may slightly reformulate the selected contextual data to render it clearer. For example, the contextual data "have you taken your medication today? No" may be turned into "medication not taken today".

The notification level may also take into account repetition of the same contextual data over time. For example, the notification level for BP data of a user which hasn't taken his or her medication two days in a row might be increased by one level each additional day.

### The correspondence database

In particular, in the present disclosure the RPM system 100 attributes a notification level to the BP data using these BP data and the selected contextual data. The selected contextual data are aimed at increasing or decreasing the notification level of the BP data compared to a non-contextual notification level, which is based only on the BP data. Selected contextual data associated with an increase of BP data value (e.g., no rest before measurement or no hypotensive medication taken) tend to decrease the notification level (since external factors temporally increase the BP data value, the high BP data value is less critical). Selected contextual data associated with an accurate assessment or a decrease of BP value tend to increase the notification level (since the BP data value is at its true value or lower, a high BP value is more critical), for a same BP value.

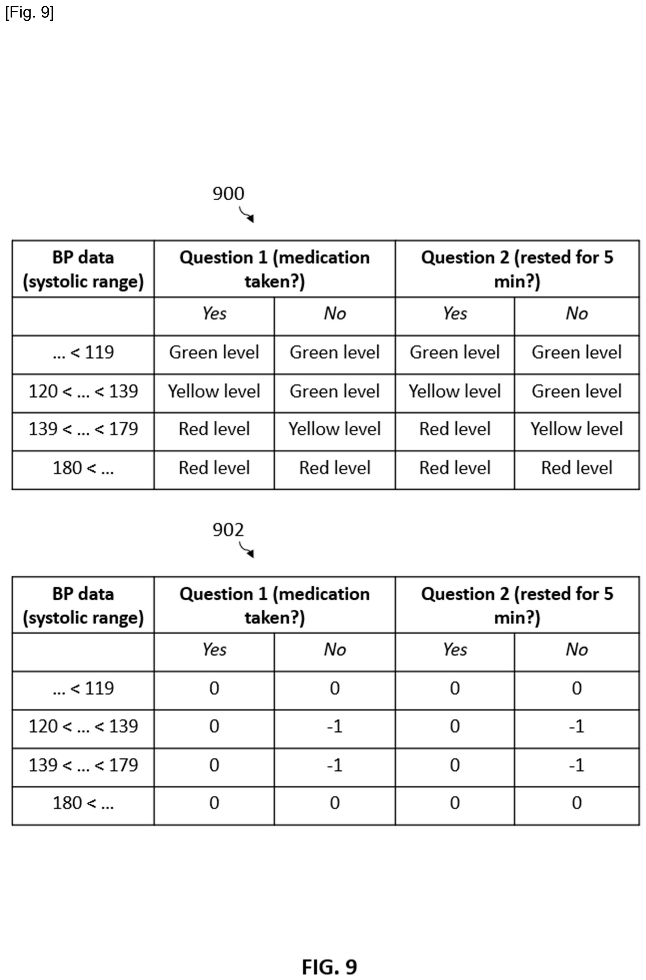

A way to determine the notification level may involve a correspondence database 900, 902 shown on Figure 9.

In an embodiment, the correspondence database 900 provides a notification level to the BP data with the selected contextual data. To that end, the correspondence database 900 takes, as inputs, the BP data (their value, for example the diastolic and systolic pressure values) and the question with the selected contextual data, and returns, as output, a notification level for the inputted BP data.

In this embodiment, the correspondence database 900 therefore includes, for at least the question displayed on the BP monitor 110a (in an embodiment, for all the question database stored in the BP monitor 110a; in another embodiment, for all the questions of the master question database), a table giving a notification level for all the combinations of BP values (e.g., ranges of BP data values) and predetermined responses for all the questions of the database.

In an embodiment, the correspondence database 902 provides a notification level variation to the BP data with the selected contextual data. To that end, the correspondence database 900 takes, as inputs, the BP data (their value, for example the diastolic and systolic pressure values) and the question with the selected contextual data, and returns, as output, a notification level variation for the inputted BP data. The variation is then to be applied to a pre-existing notification level for BP data only (without contextual data).

The correspondence database 900 may include, as entries:
- ranges for the BP data value. For example, the ranges for BP data systolic values may be: under 119 mm Hg, between 120 and 139 mm Hg, between 139 and 179 mm Hg, and above 180 mm Hg (this is only an illustrative example with no medical value whatsoever),
- the predetermined responses of each question, such as "have you taken you medication today?" Or "have you rested for 5 minutes before taking the measurement?"). At each intersection of the entries, a notification level is associated or a notification level variation is associated. The choice of notification level or notification level variation may be defined by the professional, for example through the remote monitoring interface 120. This determination may be carried out by the professional user by user, and/or disease by disease, to allow of an improved customization of the notification level. Similarly, the number of notification levels may be determined by the professional, depending on his or her own practices or on the user's needs.

For example, assuming there are only two ranges and two questions with two predetermined responses (such as YES or NO questions), then the correspondence database includes 2 ranges x 2 questions x 2 predetermined responses = 8 cells.

For example, as in correspondence database 900 of Figure 9, assuming there are only four ranges and two questions with two predetermined responses (such as YES or NO questions), then the correspondence database 900 includes 4 ranges x 2 questions x 2 predetermined responses = 16 cells.

In practice, the correspondence database 900 may then be queried to return a notification level. The query includes inputting the BP data value (such as the systolic and/diastolic value) of the BP data and the contextual data (i.e. the question and the selected predetermined responses) and returning the notification level associated with those inputs.

The size of the correspondence database directly depends on the BP data (diastolic pressure, systolic pressure, and/or heart rate), the number of questions and the number of predetermined responses for each question.

The correspondence database 900 may be stored on the BP monitor 110a, the server 130 or the remote monitoring interface 120. The attribution of the notification level to the BP data using these BP data and the selected contextual data, may be carried out by the BP monitor 110a, the server 130 or the remote monitoring interface 120. In an embodiment, since the server 130 is on the pathway of the BP data and the selected contextual data from the BP monitor 110a to the remote monitoring interface 120, the attribution of the notification level is carried out by the server 130.

### Retake feature

In some embodiments, depending on the selected contextual data, the BP monitor 110a may request the user to obtain another BP data. For example, when the selected contextual data are "have you rested for 5 min? No", then the BP monitor 110a may request so, before starting another BP measurement.

### Health condition

As presented previously, the BP monitor 110a may check a health condition for the BP data before displaying a question. In a further embodiment, the health condition is applied to other heath data of the user, collected by another measuring device of the RPM system 100, such as weight data measured by a body scale. For example, the RPM system 100 includes a body scale and the health condition is that there is a noticeable weight variation on two consecutive days.

### RPM Method

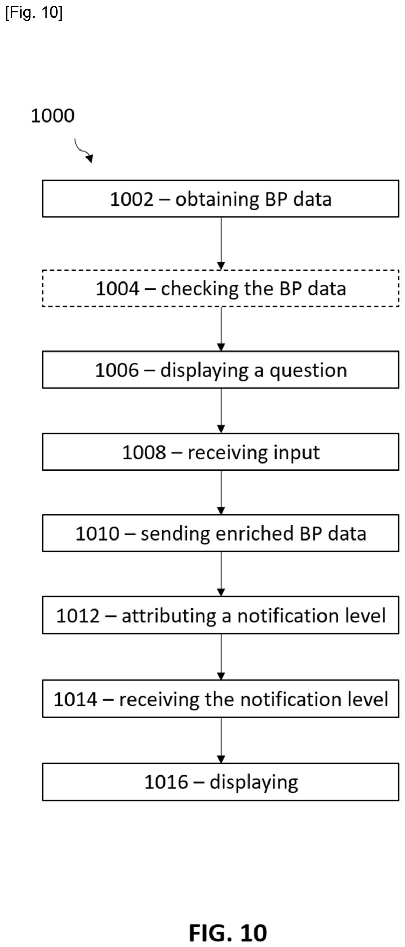

Figure 10 shows a RPM method using the RPM system 100 disclosed previously. The steps are performed either by the BP monitor 110a, the remote monitoring interface 120 or the server 130.

At 1002, the BP monitor 110a obtains BP data using the BP sensor 206. The BP data are stored in the BP monitor 110a.

At 1004, in an embodiment, the BP monitor 110a checks whether the BP data verifies a health condition. The health condition may be a range for values of the BP data, as disclosed previously. For example, the heath condition is a high systolic value of the BP data (e.g., more than 140 mm Hg).

At 1006, in response to determining that the BP data verifies the health condition, or, in the absence of such check, after (or actually before, to create a rest time for the user before taking the measurement) obtaining the BP data 1002, the BP monitor 110a displays a question on the display, along with a plurality of predetermined responses. The predetermined responses can be selected by the user with the hardware input 210. The plurality of predetermined responses associated to the question form a plurality of contextual data.

In the absence of checking 1004, displaying the question 1006 may be performed before obtaining the BP data 1002. This slows down the BP measurement process for the user and increases the quality of the measurement.

At 1008, the BP monitors 110a receives a selected predetermined response, by the user by means of the hardware input 210. The selected predetermine response (example: "YES") associated with the question (example: "have you taken your medication today?") form a selected contextual data.

At 1010, the BP monitor 110a sends the BP data and the selected contextual data to the server 130 using the wireless communication module. The BP data and the selected contextual data form a bundle, as they are associated with one another. The BP data coupled to the selected contextual data may be called "enriched BP data". In a variant, the enriched BP data may be sent directly from the BP monitor 110a to the remote monitoring interface 120.

At 1012, the server 130 attributes a notification level to the BP data using the enriched BP data, that is to say using the BP data and the selected contextual data. The attribution of the notification level 1012 is made amongst a plurality of available notification levels. More precisely, the attribution 1012 may be carried out using a query on a correspondence database 900 stored on the server 130, as disclosed previously. In a variant, the correspondence database 900 may be stored on the BP monitor 110a or the remote monitoring interface 120, and the attribution 1012 is carried out respectively by the BP monitor 110a or the remote monitoring interface 120.

At 1014, the remote monitoring interface 120 receives the enriched BP data with the attributed notification level.

At 1016, the remote monitoring interface 120 displays a display mode based on the attributed notification level.

In an embodiment, the display mode includes displaying the BP data or the enriched BP data with a specific layout associated with the label. For example, a red notification level entails a red font while a yellow notification level entails a yellow font.

In another embodiment, the display mode includes displaying a notification inbox, classified by the notification level.

In an embodiment, a display mode may include not displaying the BP data, notably when the notification level is minimum.

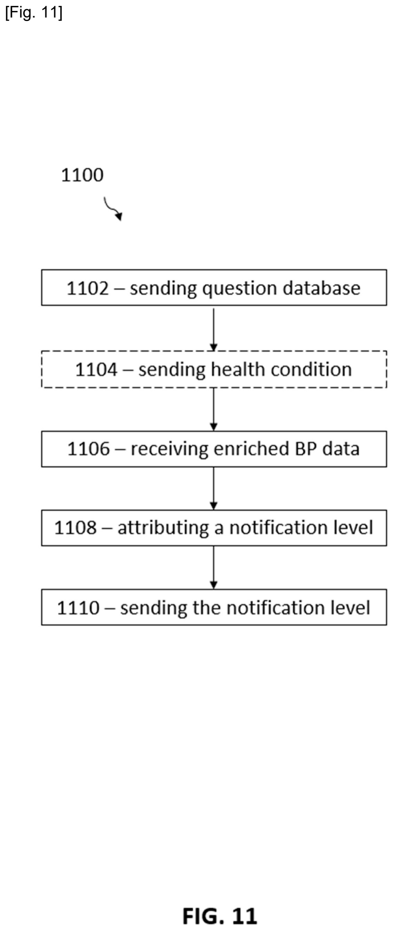

Figure 11 shows a RPM method using the RPM system 100 disclosed previously from the server 130 stand point.

At 1102, the server 130 sends to the BP monitor 110a a question database, the question database including at least one question and a plurality of predetermined responses for each question. The question and the predetermined response form contextual data.

At 1104, in an embodiment, the server 130 sends to the BP monitor 110a a health condition. The health condition is to be checked with the BP data.

At 1106, the server 130 receives from the BP monitor BP data with a predetermined response of a question. The question and the selected predetermined response form selected contextual data.

At 1108, identical to step 1012, the server 130 attributes a notification level to the BP data. To that end, the server 130 stores a correspondence database as disclosed previously.

At 1110, the server 130 sends out to the remote monitoring interface the enriched BP data along with the attributed notification level.

### Additional embodiments

In an alternative embodiment, the RPM system 100 includes the close monitoring interface of the smartphone 150 and the question is displayed on it, rather than the BP monitor 110a.

In another embodiment, the measuring device is replaced with a body scale 110b and the input hardware may include the weight sensor to select a response. Instead of monitoring the BP data, the RPM system here monitors the weight. Contextual data may involve lifestyle, meals, or response related to cardiac failure symptoms.

More generally, the physiological monitoring device comprises a user interface comprising a display and input hardware to receive a user input.

In another embodiment, the physiological measurement device 110 is a device capable of measuring temperature, blood oxygen saturation, lung or heart sounds, and/or capable of taking an electrocardiogram. An example of such device is given in document EP24217550. Particularly, this device includes a display and input hardware in a very similar fashion to that of BP monitor 110a.

## Claims

1. A remote patient monitoring, RPM, system (100) including:
• a blood pressure, BP, monitor (110a, 200), comprising:
∘ a case (202),
∘ a user interface (204) mounted on the case, comprising a display (208) and input hardware (210),
∘ a blood pressure, BP, sensor (206) configured to generate blood pressure data about the user,
∘ a wireless communication module,
• a remote monitoring interface (120), configured to run on a remote monitoring device (302), wherein the remote monitoring device (302) and the BP monitor (110, 200) are two distinct devices,
wherein the BP monitor is configured to send data to the remote monitoring interface using the wireless communication module,
wherein the RPM system (100) is configured to:
- obtain (1002) BP data using the BP sensor of the BP monitor,
- display (1006) on the display of the BP monitor (110a) a question with a plurality of predetermined responses, wherein the question and the plurality of predetermined responses form a plurality of contextual data,
- receive (1008) a predetermined response selected by the user using the input hardware of the BP monitor, wherein the question and the selected predetermined response form selected contextual data,
- attribute (1012) a notification level to the BP data using the BP data and the selected contextual data, wherein the notification level is chosen amongst a plurality of available notification levels,
- display (1016) at the remote monitoring interface a display mode based on the attributed notification level.

2. The RPM system of claim 1, wherein the plurality of available notification levels includes between 3 and 5 notification levels.

3. The RPM system of any of claims 1-2, wherein the remote monitoring interface includes a plurality of display modes, wherein each display mode includes a dedicated layout corresponding to a notification level, such that the display modes display BP data, for which contextual data are available, differently in function of the notification level.

4. The RPM system of any of claims 1-3, wherein the display mode includes a notification inbox, updated based on the attributed notification level.

5. The RPM system of any of claims 1-4, wherein the BP data are an average of several BP values.

6. The RPM system of any of claims 1-5, wherein displaying the question is performed in response to determining that the BP data verify a health condition.

7. The RPM system of claim 6, wherein the health condition is editable via the remote monitoring interface.

8. The RPM system of any of claims 1-7, wherein the display mode includes displaying the BP data along with the selected contextual data.

9. The RPM system of any of claims 1-8, further including a server (130), wherein:
- the wireless communication module of the BP monitor sends to the server the BP data and the selected contextual data,
- the remote monitoring interface receives from the server the BP data, the selected contextual data and the attributed notification level.

10. The RPM system of any of claims 1-9, wherein attributing the notification level includes:
- querying a correspondence database using the BP data and the selected contextual data, wherein the correspondence database links a plurality of notification levels or of notification level variations to ranges values for the BP data and the contextual data, wherein notification level variations are to be applied to a preexisting notification level depending only on the BP data.

11. The RPM system of claims 9 and 10, wherein the correspondence database is stored on the server and the attribution is performed by the server.

12. The RPM system of claim 10, wherein the correspondence database is stored on the BP monitor and the attribution is performed by the BP monitor.

13. The RPM system of any of claims 10-12, further including a remote monitoring device (302) configured to run the remote monitoring interface (120), wherein the correspondence database is stored in the remote monitoring device (302).

14. The RPM system of any of claims 10-13, wherein the correspondence database is editable via the remote monitoring interface.

15. A remote patient monitoring method, using a RPM system of any of claims 1-14, the method comprising:
- obtaining BP data using the BP sensor of the BP monitor,
- display on the display a question with a plurality of predetermined responses, wherein the question and the predetermined response form predetermined contextual data,
- receive a predetermined response selected by the user using the input hardware, wherein the question and the selected predetermined response form selected contextual data,
- attribute a notification level to the BP data using the BP data and the selected contextual data, wherein the notification level is chosen amongst a plurality of available notification levels,
- display at the remote monitoring interface a display mode based on the attributed notification level.
